(19) Europäisches Patentamt
European Patent Office
Office européen des brevets

(11) Numéro de publication: **0 080 941**
**B1**

(12) **FASCICULE DE BREVET EUROPEEN**

(45) Date de publication du fascicule du brevet: **17.09.86**

(21) Numéro de dépôt: **82402148.9**

(22) Date de dépôt: **25.11.82**

(51) Int. Cl.[4]: **C 07 D 487/04,** A 61 K 31/505 // (C07D487/04, 241:00, 239:00)

(54) **Nouvelles pyrimido quinoxalines et leurs sels, leur preparation, leur application à titre de médicaments et les compositions les renfermant.**

(30) Priorité: **27.11.81 GB 8135899**

(43) Date de publication de la demande:
**08.06.83 Bulletin 83/23**

(45) Mention de la délivrance du brevet:
**17.09.86 Bulletin 86/38**

(84) Etats contractants désignés:
**AT BE CH DE FR IT LI LU NL SE**

(56) Documents cités:
**FR-A-2 265 389**

(73) Titulaire: **ROUSSEL-UCLAF**
**35, boulevard des Invalides**
**F-75007 Paris (FR)**

(72) Inventeur: **Kennewell, Peter David**
**10 St. Helen's View**
**Swindon Wiltshire (GB)**
Inventeur: **Kay, David Paul**
**4 Church Path Purton**
**Swindon Wiltshire (GB)**

(74) Mandataire: **Vieillefosse, Jean-Claude et al**
**Département des Brevets ROUSSEL UCLAF B.P. no. 9**
**F-93230 Romainville (FR)**

**Le dossier contient des informations techniques présentées postérieurement au dépôt de la demande et ne figurant pas dans le présent fascicule**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 080 941 B1**

**Description**

La présente invention concerne de nouvelles pyrimido quinoxalines et leurs sels, ainsi que le procédé de préparation, l'application à titre de médicaments de ces nouveaux produits et les compositions les renfermant.

La demande de brevet FR—A—2265389 décrit d'autres dérivés trycycliques à savoir des pyrimido/1,2a/ quinoléines présentant des propriétés antiallergiques.

Les produits de la présente invention se distinguent des produits de l'art antérieur par le fait qu'ils sont particulièrment actifs par voie orale.

L'invention a pour objet de nouvelles pyrimido-quinoxalines, ainsi que leurs sels, caractérisées en ce qu'elles répondant à la formule générale I

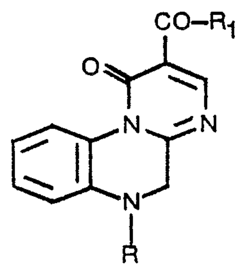

(I)

dans laquelle R représente un atome d'hydrogène, un radical alcoyle renfermant de 1 à 5 atomes de carbone, un radical —CO—$R_A$ dans lequel $R_A$ représente un groupement alcoyle, linéaire ou ramifé, renfermant de 1 à 6 atomes de carbone, un radical cycloalcoyle renfermant de 3 à 6 atomes de carbone, un radical phényle éventuellement substitué par un ou plusieurs atomes d'halogène, par un ou plusieurs radicaux alcoxy renfermant de 1 à 3 atomes de carbone ou par un ou plusieurs radicaux nitro, ou $R_A$ représente un radical alkoxy renfermant de 1 à 5 atomes de carbone, ou R représente un groupement —CHO ou —CO—NH—$C_6H_5$,

$R_1$ représente un radical alcoxy renfermant de 1 à 5 atomes de carbone.

Dans la formule générale I et dans ce qui suit, le terme radical alcoyle renfermant de 1 à 5 atomes de carbone désigne, par exemple, un radical méthyle, éthyle, propyle, isopropyle, butyle, isobutyle, terbutyle ou pentyle; le terme radical alcoyle linéaire ou ramifé, renfermant de 1 à 6 atomes de carbone désigne par exemple un radical méthyle, éthyle, propyle, isopropyle, butyle, isobutyle, terbutyle, pentyle, hexyle; le terme radical cycloalcoyle renfermant de 3 à 6 atomes de carbone désigne, par exemple, un radical cyclopropyle ou cyclopentyle; le terme radical phényle éventuellement substitué par un ou plusieurs atomes d'halogène désigne, par exemple, un radical p-chlorophényle, o-chlorophényle, m-chlorophényle ou m-p-dichlorophényle; le terme radical alcoxy renfermant de 1 à 5 atomes de carbone désigne, par exemple, un radical méthoxy, éthoxy, propoxy ou butoxy.

Les produits de formule I présentent un caractère basique. Ils peuvent advantageusement former des sels d'addition avec les acides minéraux ou organiques; ces sels peuvent être par exemple les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfoniques, tels que l'acide méthane sulfonique et arylsulfoniques, tels que l'acide benzène sulfonique.

Parmi les produits objet de l'invention, on peut citre notamment les dérivés répondant à la formule I ci-dessus, ainsi que leurs sels, caractérisés en ce que dans ladite formule I R a la signification déjà indiquée et $R_1$ représente un radical éthoxy.

Parmi les produits objet de l'invention, on retient plus particulièrement les dérivés répondant à la formule I ci-dessus, ainsi que leurs sels, caracfrisés en ce que dans ladite formule I, R représente un atome d'hydrogène, un radical méthyle, un radical —CO—$R_A$ dans lequel $R_A$ représente un groupement méthyle, éthyle, isopropyle, cyclopropyle, cyclopentyle, phényle, p-chlorophényle ou éthoxy, ou R représente un groupement —CHO ou —CO—NH—$C_6H_5$.

Parmi ces derniers, on peut citer tout particulièrement:

le 1-oxo 5,6-dihydro-1H-pyrimido/1,2-a/quinoxalin-2-carboxylate d'éthyle,

le 6-benzoyl-5,6-dihydro-1H-pyrimido/1,2-a/quinoxalin-2-carboxylate d'éthyle,

le 1-oxo 5,6-dihydro-1H-pyrimido/1,2-a/quinoxalin-2,6-dicarboxylate d'éthyle, ainsi que leurs sels.

L'invention a également pour objet un procédé de préparation des produits répondant à la formule

générale I, tels que définis ci-dessus, ainsi que de leurs sels caractérisé en ce que l'on réduit un produit de formule II:

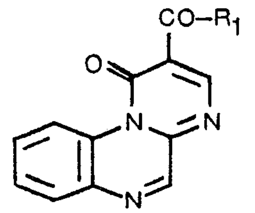

(II)

dans laquelle $R_1$ a la signification déjà indiquée, pour obtenir un produit de formule $I_a$:

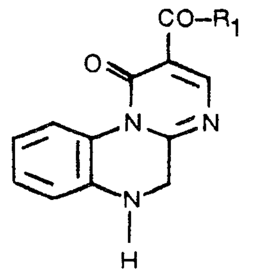

($I_a$)

dans laquelle $R_1$ a la signification déjà indiquée et que,

*soit* on isole et, si désiré, salifie ledit produit de formule $I_a$ ainsi obtenu,

*soit* on fait régir ledit produit de formule $I_a$ avec un réactif permettant d'introduire le radical R, pour obtenir un produit de formule $I_b$:

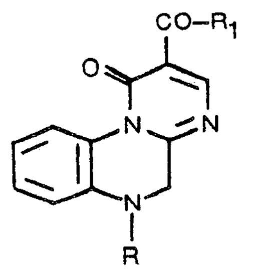

($I_b$)

dans laquelle $R_1$ a la signification déjà indiquée et R a la signification déjà indiquée, à l'exception d'un atome d'hydrogène, que l'on peut salifier si désiré.

Dans des conditions préférentielles de mise en oeuvre l'invention, le procédé de préparation ci-dessus décrit est caractérisé en ce que:

a) la réduction du produit de formule II est effectuée au moyen de l'hydrogène en présence d'un catalyseur tel que le palladium,

b) le réactif permettant d'introdaire le radical R dans le cas où R représente un radical alcoyle ou un radical —CO—$R_A$ est constitué par un halogénure de ce radical R, de préférence un chlorure.

c) le réactif permettant d'introduire le radical R dans le cas où R représente un groupement —CHO est constitué par de l'acide formique en présence de carbonyl-diimidazole.

d) le réactif permettant d'introduire le radical R dans le cas où représente un groupement —CO—NH—$C_6H_5$ est l'isocyanate de phényle,

e) le réactif permettant d'introduire le radical R dans le cas où R représente un radical méthyle est constitué par de l'aldéhyde formique et l'action de ce dernier est suivie par un réduction.

Les sels peuvent être préparés en faisant réagir en proportions sensiblement stoechiométriques un acide minéral ou organique avec un produit de formule I.

Les produits objet de la présente invention possèdent de très intéressantes propriétés pharmacologiques; ils sont doués notamment de remarquables propriétés antiallergiques.

Ces propriétés sont illustrées plus loin dans la partie expérimentale.

Ces propriétés justifient l'utilisation des nouvelles pyrimidoquinoxalines, objet de la présente invention, ainsi que de leurs sels, pharmaceutiquement acceptables, à titre de médicaments.

La présente demande a ainsi également pour objet l'application, à titre de médicaments, des nouvelles pyrimido-quinoxalines telles que définies par la formule I, ainsi que de leurs sels d'addition avec les acides pharmaceutiquement acceptables.

Parmi les médicaments objet de l'invention, on retient de préférence les médicaments caracteŕisé en ce qu'ils sont constitués par les dérivés répondant à la formule I dans laquelle R a la signification déjà

indiquée et $R_1$ représente un radical éthoxy, ainsi que par leurs sels d'addition avec les acides pharmaceutiquement acceptables.

Parmi ceux-ci, on retient notamment les médicaments caractérisés en ce qu'ils sont constitués par les dérivés répondant à la formule I dans laquell R représente un atome d'hydrogène, un radical, méthyle, un radical —CO—$R_A$ dans lequel $R_A$ représente un groupement méthyle, éthyle, isopropyle, cyclopropyle, cyclopentyle, phényle, p-chlorophényle ou éthoxy, ou R représente un groupement —CHO ou —CO—NH—$C_6H_5$, ainsi que par leurs sels d'addition avec les acides pharmaceutiquement acceptables.

Parmi ces derniers, on retient tout particulièrement les produits dont les noms suivent:

le 1-oxo 5,6-dihydro-1H-pyrimido/1,2-a/quinoxalin-2-carboxylate d'éthyle,

le 6-benzoyl-1-oxo-5,6-dihydro-1H-pyrimido/1,2-a/quinoxalin-2-carboxylate d'éthyle,

le 1-oxo 5,6-dihydro-1H-pyrimido/1,2-a/quinoxalin-2,6-dicarboxylate d'éthyle, ainsi que leurs sels d'addition avec les acides pharmaceutiquement acceptables.

Ces médicaments trouvent, par exemple, leur emploi dans le traitement de l'asthme allergique et des bronchites asthmatiformes d'origine allergique.

La dose usuelle, variable selon le produit utilisé, le sujet traité et l'affection en cause peut être, par exemple, de 1 mg à 1000 mg par jour, par voie orale chez l'homme.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être par exemple, solides ou liquides et se présenter sous les formes pharmaceutiques couramment utilisées en médecine humaine, comme, par exemple, les comprimés, simples ou dragéifiés, les gélules, les granulés, les suppositoires, les sirops, les aérosols, les crèmes, les pommades, les préparations injectables, elles sont préparées selon les méthodes usuelles. Le ou les principes actifs peuvent y être incorporés à des excipients habituellement employés dans ces compositions pharmaceutiques, tels que la talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Le produit de formule II dans laquelle $R_1$ représente un radical éthoxy a notamment été décrit par I. Hermecz et al dans J. Chem. Soc. Perkin I, 789 (1977).

Les autres produits de formule II dans laquelle $R_1$ représente un radical méthoxy ou un radical alcoxy renfermant de 3 à 5 atomes de carbone peuvent être préparés par un procédé analogue.

Les exemples suivants illustrent l'invention sans toutefois la limiter.

## Exemple 1

5,6-dihydro 1-oxo-1H-pyrimido/1,2-a/quinoxaline-2-carboxylate d'éthyle.,

On dissout 5 g de 1-oxo-1H-pyrimido/1,2-a/quinoxaline-2-carboxylate d'éthyle [I. Hermecz, Chem. Soc. Perkin I, 789 (1977)] dans 400 cm3 d'acétate d'éthyle, puis ajoute 1 cm3 d'acide chlorhydrique concentré et 300 mg de palladium sur charbon à 5%. On place le mélange sous agitation, sous atmosphère d'hydrogène pendant 5 heures à 20°C. Lorsque la réaction est terminée, on basifie de mélange par addition d'une solution de bicarbonate de sodium et filtre. On décante, sèche la phase organique et évapore le solvant à 35°C. On obtient 4,2 g de produit attendu.

F = 170°C.

## Exemple 2

5,6-dihydro 1-oxo-1H-pyrimido/1,2-a/quinoxaline-2,6-dicarboxylate d'éthyle .

On fait passer un courant d'azote pendant 10 minutes dans 4 cm3 de diméthylformamide anhydre, puis ajoute 0,150 g de chloroformiate d'éthyle et 0,280 g de produit obtenu à l'exemple 1. On ferme hermétiquement et maintient une nuit à 20°C, puis verse dans l'eau et extrait à l'acétate d'éthyle. On sèche la phase organique, évapore le solvant et chromatographie la résidu sur silice en éluant à l'acétate d'éthyle. On obtient 0,14 g de produit attendu.

F = 140—142°C.

## Exemple 3

6-acétyl 5,6-dihydro 1-oxo 1H-pyrimido/1,2-a/quinoxaline-2-carboxylate d'éthyle.

On agite sous atmosphère inerte un mélange de 0,65 g de chlorure d'acétyle, 1,5 g de carbonate de sodium et 70 cm3 de chlorure de méthylène, tout en ajoutant 1,5 g de produit obtenu à l'exemple 1. On maintient l'agitation pendant 4 heures, lave la solution à l'eau, sèche et évapore le solvant. On obtient 1,45 g produit attendu.

F = 152—155°C.

Exemples 4 à 12

En utilisant une méthode analogue à celle de l'exemple 3 et un utilisant au départ un chlorure correspondant Cl—R, dans lequel R est défini comme dans le tableau ci-après, on a préparé les produits attendus.

| Ex | $R_1$ | R | F°C | Formule brute | Analyse Calculé C % | H % | N % | Analyse Trouvé C % | H % | N % |
|---|---|---|---|---|---|---|---|---|---|---|
| 1 | $OC_2H_5$ | H | ~170 (d) | $C_{14}H_{13}N_3O_3$ | 61.99 | 4.83 | 15.49 | 62.17 | 4.85 | 15.38 |
| 2 | $OC_2H_5$ | $CO_2Et$ | 140–2 | $C_{17}H_{17}N_3O_5$ | 59.47 | 4.99 | 12.24 | 59.51 | 5.03 | 11.97 |
| 3 | $OC_2H_5$ | $COCH_3$ | 152–5 | $C_{16}H_{15}N_3O_4$ | 61.34 | 4.83 | 13.41 | 61.08 | 4.83 | 13.31 |
| 4 | $OC_2H_5$ | $COCHMe_2$ | 123–4 | $C_{18}H_{19}N_3O_4$ | 63.33 | 5.61 | 12.31 | 63.46 | 5.68 | 12.21 |
| 5 | $OC_2H_5$ | 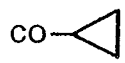 | 184–6 | $C_{18}H_{17}N_3O_4$ | 63.71 | 5.05 | 12.38 | 63.48 | 5.09 | 12.19 |
| 6 | $OC_2H_5$ | COPh | 168–70 | $C_{21}H_{17}N_3O_4$ | 67.19 | 4.56 | 11.19 | 66.90 | 4.59 | 11.11 |
| 7 | $OC_2H_5$ | COEt | 182–4 | $C_{17}H_{17}N_3O_4$ | 62.38 | 5.23 | 12.84 | 62.14 | 5.22 | 12.66 |
| 8 | $OC_2H_5$ | 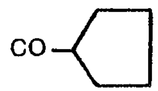 | 92–5 | $C_{20}H_{21}N_3O_4$ | 65.38 | 5.76 | 11.44 | 65.08 | 5.86 | 11.18 |
| 9 | $OC_2H_5$ | 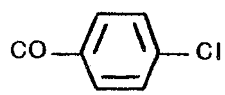 | 140–2 | | | | | | | |
| 10 | $OC_2H_5$ | 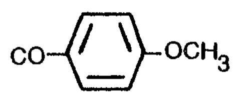 | 105–7 | | | | | | | |
| 11 | $OC_2H_5$ | 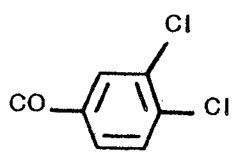 | 120–2 | $C_{21}H_{15}Cl_2N_3O_4$ | 56.77 | 3.40 | 9.46 | 56.58 | 3.41 | 9.46 |
| 12 | $OC_2H_5$ | 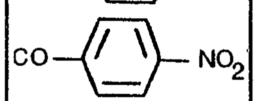 | 134–5 | | | | | | | |
| 13 | $OC_2H_5$ | CHO | 172–4 | $C_{15}H_{13}N_3O_4$ | 60.26 | 4.38 | 14.04 | 60.31 | 4.44 | 13.91 |
| 14 | $OC_2H_5$ | CONHPh | 188–90 | $C_{21}H_{18}N_4O_4$ | 64.61 | 4.65 | 14.35 | 64.66 | 4.72 | 14.24 |
| 15 | $OC_2H_5$ | $CH_3$ | 111–4 | $C_{15}H_{15}N_3O_3$ | 63.15 | 5.30 | 14.73 | 62.75 | 5.35 | 14.53 |

Exemple 13

5,6-dihydro 6-formyl 1H-pyrimido/1,2-a/quinoxaline-2-carboxylate d'éthyle.

On ajoute à 20°C, sous gaz inerte, 0,1 g de carbonyldiimidazole à une solution de 0,7 g d'acide formique à 98% dans 70 cm3 de chlorure de méthylène. Après 30 minutes, on ajoute 1,5 g de produit obtenu à l'exemple 1. On agite pendant 6 heures, verse dans une solution de bicarbonate de sodium et extrait à l'acétate d'éthyle. On sèche la phase organique, la traite au charbon actif et évapore le solvant. On recristallise le résidu dans l'acétate d'éthyle et obtient 1,1 g de produit attendu.

F = 172—174°C.

Exemple 14

5,6-dihydro 1-oxo 6-phénylecarbamoyl 1H-pyrimido/1,2-a/quinoxaline-2-carboxylate d'éthyle.

On porte au reflux sous agitation, sous gas inerte, pendant 15 heures, un mélange de 1,5 g de produit obtenu à l'exemple 1, 0,8 cm3 d'isocyanate de phényle et 70 cm3 de toluène anhydre. On refroidit, filtre et sèche les cristaux formés. On obtient 1,47 g de produit attendu.

F = 188—190°C.

Exemple 15

5,6-dihydro 6-méthyl 1-oxo 1H-pyrimido/1,2-a/quinoxaline-2-carboxylate d'éthyle.

On mélange 1,5 de produit obtenu à l'exemple 1, 200 cm3 d'éthanol et 0,5 g d'acide chlorhydrique concentré. On ajoute 5 cm3 de formaldéhyde à 37% dans l'eau et 0,2 g de palladium sur charbon à 5%. On place sous agitation, sous atmosphère d'hydrogène, pendant 4 heures. On filtre, concentre, ajoute un solution 0,6 g de bicarbonate de sodium dans 60 cm3 d'eau et extrait à l'acétate d'éthyle. On sèche la phase organique, évapore le solvant et chromatographie le résidu sur silice en éluant au chlorure de méthylène à 1% méthanol. On obtient 0,8 g de produit attendu.

F = 111—114°C.

Exemple 16

Composition pharmaceutique

On a préparé des comprimés répondant à la formule:

| | | |
|---|---|---|
| Produit de l'exemple 1 | | 0,015 g |
| Excipient | q.s.p. | 0,1 g. |

(Détail de l'excipient: lactose, talc, amidon, stéarate de magnésium).

Exemple 17

Composition pharmaceutiquement

On a préparé un aérosol délivrant par dose:

| | |
|---|---|
| Produit de l'exemple 1 | 0,002 g |
| Emulsifiant | 0,00015 g |
| Propulseur | 0.050 g |

Etude pharmacologique

Anaphylaxie cutanée passive (ACP) expérimentale chez le rat.

On induit une anaphylaxie cutanée passive chez des rats mâles Wistar pesant 180—200 g. Les rats ont été sensibilisés par 4 injections intradermiques, au niveau de leur dos préalablement rasé, afin de produire une réaction cutanée passive faisant intervenir des anticorps IgG. (Sensibilisation de 4 heures, par antisérum chauffé pendant 1 heure à 56°C).

Le traitement avec l'antigène est effectué ensuite: 1 mg d'ovalbumine est injecté par voie intraveineuse, en même temps que 0,5 cm3 d'une solution à 1% de bleu d'Evans et 30 minutes après, les animaux sont sacrifiés et pour chaque point bleu observé à la surface interne de la peau, une notation est donnée en tenant compte de la surface et de la gravité.

Le pourcentage d'inhibition observé après l'administration orale des composés à tester est rapporté dans le tableau ci-après.

| Composé de l'exemple | % inhibition de la réaction cutanée | | |
|---|---|---|---|
| | 0,1 mg/Kg | 1 mg/Kg | 10 mg/Kg |
| 1 | | 23,0 | 51,0 |
| 2 | | 31,0 | 34,9 |
| 3 | | 18,5 | 33,3 |
| 4 | 4,1 | 16,0 | 8,9 |
| 5 | | 23,0 | 33,3 |
| 6 | | 23,0 | 43,0 |
| 7 | | 8,2 | 5,8 |
| 8 | | 5,4 | 14,8 |
| 13 | 0,6 | 4,1 | 12,4 |
| 14 | | 17,6 | 15,2 |
| 15 | 7,7 | 7,7 | 30,2 |

**Revendications pour les Etats Contractants: BF CH DE FR IT LI LU NL SE**

1. Pyrimido-quinoxalines, ainsi que leurs sels, caractérisés en ce qu'elles répondent à la formule générale I:

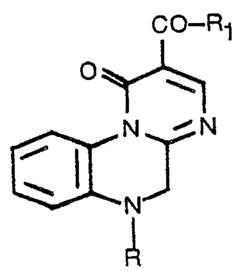

(I)

dans laquelle R représente un atome d'hydrogène, un radical alcoyle renfermant de 1 à 5 atomes de carbone, un radical —CO—$R_A$ dans lequel $R_A$ représente un groupement alcoyle, linéaire ou ramifé, renfermant de 1 à 6 atomes de carbone, un radical cycloalcoyle renfermant de 3 à 6 atomes de carbone, un radical phényle éventuellement substitué par un ou plusieurs atomes d'halogène, ou par un ou plusieurs radicaux alcoxy renfermant de 1 à 3 atomes de carbone ou par un ou plusieurs radicaux nitro, ou $R_A$ représente un radical alkoxy renfermant de 1 à 5 atomes de carbone, ou R représente un groupement —CHO ou —CO—NH—$C_6H_5$,

$R_1$ représente un radical alcoxy renfermant de 1 à 5 atomes de carbone.

2. Dérivés répondant à la formule générale I de la revendication 1, ainsi que leurs sels, caractérisés en ce que dans ladite formule I R a la signification déjà indiquée et $R_1$ représente un radical éthoxy.

3. Dérivés répondant à la formule générale I de la revendication 1, ainsi que leurs sels, caractérisés en ce que dans ladite formule I R représente un atome d'hydrogène, un radical méthyle, un radical —CO—$R_A$ dans lequel $R_A$ représente un groupement méthyle, éthyle, isopropyle, cyclopropyle, cyclopentyle, phényle, p-chlorophényle ou éthoxy, ou R représente un groupement —CHO ou —CO—NH—$C_6H_5$.

4. Le 1-oxo 5,6-dihydro-1H-pyrimido/1,2-a/quinoxalin-2-carboxylate d'éthyle, ainsi que ses sels.

5. Le 6-benzoyl-1-oxo-5,6-dihydro-1H-pyrimido/1,2-a/quinoxalin-2-carboxylate d'éthyle, ainsi que ses sels.

6. Le 1-oxo 5,6-dihydro-1H-pyrimido/1,2-a/quinoxalin-2,6-dicarboxylate d'éthyle, ainsi que ses sels.

7. Procédé de préparation des produits répondant à la formule générale I de la revendication 1, ainsi que de leurs sels, caractérisé en ce que l'on réduit produit de formule II:

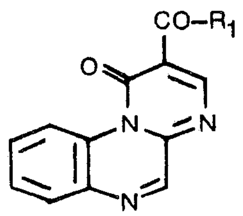

(II)

dans laquelle $R_1$ a la signification déjà indiquée, pour obtenir un produit de formule $I_a$:

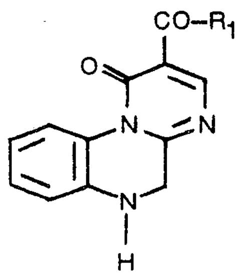

($I_a$)

correspondant à un produit de formule I,
dans laquelle $R_1$ signification déjà indiquée et R représente un atome d'hydrogène, et, que,

*soit* on isole et, si désiré, salifie ledit produit de formule $I_a$ ainsi obtenu,

*soit* on fait régir ledit produit de formule $I_a$ avec un réactif permettant d'introduire le radical R, pour différent d'un atome d'hydrogène, pour obtenir un produit de formule $I_b$:

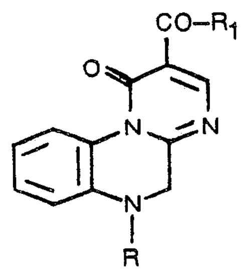

($I_b$)

correspondant à un produit de formule I,
dans laquelle $R_1$ signification déjà indiquée et R a la signification déjà indiquée, à l'exception d'une atome d'hydrogène, que l'on peut salifier, si désiré.

8. Procédé selon la revendication 7, caractérisé en ce que:

a) la réduction du produit de formule II est effectuée au moyen de l'hydrogène en présence d'un catalyseur tel que le palladium,

b) le réactif permettant d'introdaire le radical R dans le cas où R représente un radical alcoyle ou un radical —CO—$R_A$ est constitué par un halogénure de ce radical R, de préférence un chlorure,

c) le réactif permettant d'introduire le radical R dans le cas où R représente un groupement —CHO est constitué par de l'acide formique en présence de carbonyl-diimidazole,

d) le réactif permettant d'introduire le radical R dans le cas où R représente un groupement —CO—NH—$C_6H_5$ est l'isocyanate de phényle,

e) le réactif permettant d'introduire le radical R dans le cas où R représente un radical méthyle est constitué par de l'aldéhyde formique et l'action de ce dernier est suivie par un réduction.

9. Médicaments, caractérisés en ce qu'ils sont constitués par les nouveaux dérivés tels que définis par la formule I de la revendication 1, ainsi que par leurs sels d'addition avec les acides pharmaceutiquement acceptables.

10. Médicaments, caractérisés en que qu'ils sont constitués par les nouveaux dérivés tels que définis à l'une quelconque des revendications 2 à 6, ainsi que par leurs sels d'addition avec les acides pharmaceutiquement acceptables.

11. Compositions pharmaceutiques, caractérisées en ce qu'elles renferment à titre de principe actif, l'un au moins des médicaments tels que définis à l'une quelconque des revendications 9 ou 10.

**Revendications pour l'Etat contractant: AT**

1. Procédé de préparation de nouvelles pyrimido quinoxalines, ainsi que de leurs sels, répondant à la formule générale I:

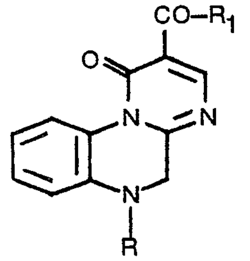

(I)

dans laquelle R représente un atome d'hydrogène, un radical alcoyle renfermant de 1 à 5 atomes de carbone, un radical —CO—$R_A$ dans lequel $R_A$ représente un groupement alcoyle, linéaire ou ramifié, renfermant de 1 à 6 atomes de carbone, un radical cycloalcoyle renfermant de 3 à 6 atomes de carbone, un radical phényle éventuellement substitué par un ou plusieurs atomes d'halogène, par un ou plusieurs radicaux alcoxy renfermant de 1 à 3 atomes de carbone ou par un ou plusieurs radicaux nitro, ou $R_A$ représente un radical alkoxy renfermant de 1 à 5 atomes de carbone, ou R représente un groupement —CHO ou —CO—NH—$C_6H_5$,

$R_1$ représente un radical alcoyloxy renfermant de 1 à 5 atomes de carbone, caractérisé en ce que l'on réduit un produit de formule II:

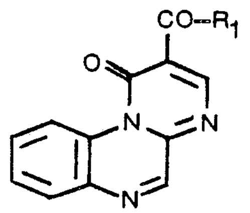

(II)

dans laquelle $R_1$ a la signification déjà indiquée, pour obtenir un produit de formule $I_a$: correspondant à un produit de formule I,
dans laquelle $R_1$ signification déjà indiquée et R représente un atome d'hydrogène, et, que,

*soit* on isole et, si désiré, salifie ledit produit de formule $I_a$ ainsi obtenu,

*soit* on fait régir ledit produit de formule $I_a$ avec un réactif permettant d'introduire le radical R, différent d'un atome d'hydrogène, pour obtenir un produit de formule $I_b$:

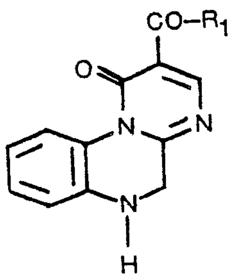

($I_a$)

correspondant à un produit de formule I,
dans laquelle $R_1$ signification déjà indiquée et R a la signification déjà indiquée, à l'exception d'une atome d'hydrogène, que l'on peut salifier, si désiré.

8. Procédé selon la revendication 7, caractérisé en ce que:

a) la réduction du produit de formule II est effectuée au moyen de l'hydrogène en présence d'un catalyseur tel que le palladium,

b) le réactif permettant d'introduire le radical R dans le cas où R représente un radical alcoyle ou un radical —CO—$R_A$ est constitué par un halogénure de ce radical R, de préférence un chlorure,

c) le réactif permettant d'introduire le radical R dans le cas où R représente un groupement —CHO est constitué par de l'acide formique en présence de carbonyl-diimidazole,

d) le réactif permettant d'introduire le radical R dans le cas où R représente un groupement —CO—NH—$C_6H_5$ est l'isocyanate de phényle,

e) le réactif permettant d'introduire le radical R dans le cas où R représente un radical méthyle est constitué par de l'aldéhyde formique et l'action de ce dernier est suivie par un réduction.

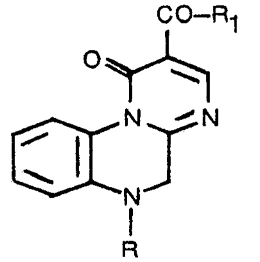

($I_b$)

3. Procédé selon la revendication 1 ou 2, caractérisé en ce que l'on utilise au départ un produit de formule II dans laquelle $R_1$ représente un radical éthoxy.

4. Procédé selon la revendication 1, 2 ou 3, caractérisé en ce que le réactif permettant d'introduire le radical R est choisi de telle sorte que l'on introduise un radical méthyle, un radical —CO—$R_A$ dans lequel $R_A$ représente un groupement méthyle, éthyle, isopropyle, cyclopropyle, cyclopentyle, phényle, p-chlorophényle ou éthoxy, ou un groupement —CHO ou —CO—NH—$C_6H_5$.

5. Procédé selon l'une quelconque des revendications 1 à 4, caractérisé en ce que l'on prépare le 1-oxo 5,6-dihydro-1H-pyrimido/1,2-a/quinoxalin-2-carboxylate d'éthyle, ainsi que ses sels.

6. Procédé selon l'une quelconque des revendications 1 à 4, caractérisé en ce que l'on prépare le 6-benzoyl 1-oxo 5,6-dihydro-1H-pyrimido/1,2-a/quinoxalin-2-carboxylate d'éthyle, ainsi que ses sels.

7. Procédé selon l'une quelconque des revendications 1 à 4, caractérisé en ce que l'on prépare le 1-oxo 5,6-dihydro-1H-pyrimido/1,2-a/quinoxalin-2,6-dicarboxylate d'éthyle, ainsi que ses sels.

**Patentansprüche für die Vertragsstaaten: BE CH DE FR IT LI LU NL SE**

1. Pyrimidochinoxaline sowie deren Salze, dadurch gekennzeichnet, daß sie der allgemeinen Formel I

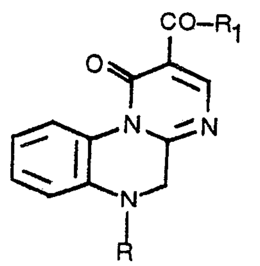

(I)

entsprechen, worin R ein Wasserstoffatom, einen Alkylrest mit 1 bis 5 Kohlenstoffatomen, einen Rest —CO—$R_A$ bedeutet, worin $R_A$ eine lineare oder verzweigte Alkylgruppe mit 1 bis 6 Kohlenstoffatomen, einen Cycloalkylrest mit 3 bis 6 Kohlenstoffatomen, einen gegebenenfalls durch eine oder mehrere Halogenatome oder durch einen oder mehrere Alkoxyreste mit 1 bis 3 Kohlenstoffatomen oder durch ein oder mehrere Nitrogruppen substituierten Phenylrest bedeutet, oder $R_A$ eine Alkoxygruppe mit 1 bis 5 Kohlenstoffatomen darstellt, oder R eine Gruppe —CHO oder —CO—NH—$C_6H_5$ bedeutet und $R_1$ einen Alkyoxyrest mit 1 bis 5 Kohlenstoffatomen bedeutet.

2. Der allgemeinen Formel I des Anspruchs 1 entsprechende Derivate sowie deren Salze, dadurch gekennzeichnet, daß in der Formel I R die angegebene Bedeutung besitzt und $R_1$ einen Ethoxyrest bedeutet.

3. Der allgemeinen Formel I des Anspruchs 1 entsprechende Derivate sowie deren Salze, dadurch gekennzeichnet, daß in der Formel I R ein Wasserstoffatom einen Methylrest, einen Rest-CO—$R_A$, worin $R_A$ eine Methyl-, Ethyl-, Isopropyl-, Cyclopropyl-, Cyclopentyl-, Phenyl-, p-Chlorphenyl- oder Ethoxygruppe bedeutet, darstellt oder R eine Gruppe —CHO oder —CO—NH—$C_6H_5$ bedeutet.

4. 1-Oxo-5,6-dihydro-1H-pyrimido[1,2-a]chinoxalin-2-carbonsäure-ethylester sowie dessen Salze.

5. 6-Benzoyl-1-oxo-5,6-dihydro-1H-pyrimido[1,2-a]chinoxalin-2-carbonsäure-ethylester sowie dessen Salze.

6. 1-Oxo-5,6-dihydro-1H-pyrimido[1,2-a]chinoxalin-2,6-dicarbonsäure-ethylester sowie dessen Salze.

7. Verfahren zur Herstellung der Produkte de allgemeinen Formel I des Anspruchs 1 sowie von deren Salzen, dadurch gekennzeichnet, daß man ein Produkt der Formel II

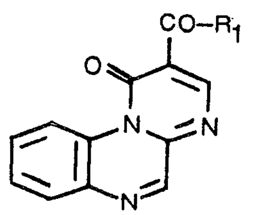

(II)

worin $R_1$ die angegebene Bedeuten besitzt, reduziert, um ein Produkt der Formel $I_a$

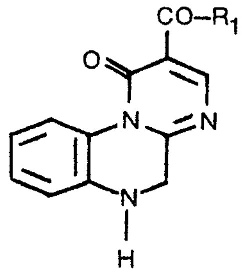

($I_a$)

entsprechend einem Produkt der Formel I, worin $R_1$ die angegebene Bedeutung besitzt und R ein Wasserstoffatom bedeutet, zu erhalten, und

*entweder* man das so erhaltene Produkt der Formel $I_a$ isoliert und gewünschtenfalls in ein Salz überführt

*oder* das Produkt der Formel $I_a$ mit einem Reagens umsetzt, das es ermöglicht, den Rest R, der von einem Wasserstoffatom verschieden ist, einzuführen, um ein Produkt der Formel $I_b$

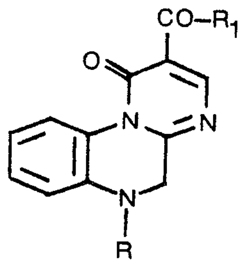

($I_b$)

entsprechend einem Produkt der Formel I, worin $R_1$ die angegebene Bedeutung besitzt und R die angegebene Bedeuten mit Ausnahme eines Wasserstoffatoms besitzt, zu erhalten, das man gewünschtenfalls in ein Salz überführen kann.

8. Verfahren gemäß Anspruch 7, dadurch gekennzeichnet, daß

(a) die Reduktion des Produkts der Formel II mit Hilfe von Wasserstoff in Gegenwart eines Katalysators, wie Palladium, erfolgt,

(b) das Reagens, das es ermöglicht, den Rest R einzuführen, in dem Fall, bei dem R einen Alkylrest oder einen Rest $-CO-R_A$ bedeutet, aus einem Halogenid des Restes R, vorzugsweise einem Chlorid, besteht,

(c) das Reagens, das es ermöglicht, den Rest R einzuführen, in dem Fall, bei dem R eine Gruppe $-CHO$ bedeutet, aus Ameisensäure in Gegenwart von Carbonyldiimidazol besteht,

(d) das Reagens, das es ermöglicht, den Rest R einzuführen, in dem Fall, bei dem R eine Gruppe $-CO-NH-C_6H_5$ bedeutet, Phenylisocyanat ist,

(e) das Reagens, das es ermöglicht, den Rest R einzurühren, in dem Fall, bei dem R einen Methylrest bedeutet, Formaldehyd ist und der Umsetzung dieses letzteren sich eine Reduktion anschließt.

9. Arzneimittel, dadurch gekennzeichnet, daß sie aus den neuen Derivaten der Formel I gemäß Anspruch 1 sowie aus deren Additionssalzen mit pharmazeutisch verträglichen Säuren bestehen.

10. Arzneimittel, dadurch gekennzeichnet, daß sie aus den neuen Derivaten gemäß einem der Ansprüche 2 bis 6 sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel gemäß einem der Ansprüche 9 oder 10 enthalten.

**Patentansprüche für den Vertragsstaat: AT**

1. Verfahren zur Herstellung neuer Pyrimidochinoxaline sowie von deren Salzen der allgemeinen Formel I

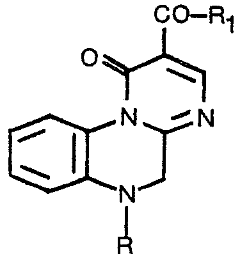

(I)

worin R ein Wasserstoffatom, einen Alkylrest mit 1 bis 5 Kohlenstoffatomen, einen Rest $-CO-R_A$

bedeutet, worin $R_A$ eine lineare oder verzweigte Alkylgruppe mit 1 bis 6 Kohlenstoffatomen, einen Cycloalkylrest mit 3 bis 6 Kohlenstoffatomen, einen gegebenenfalls durch eine oder mehrere Halogenatome oder durch einen oder mehrere Alkoxyreste mit 1 bis 3 Kohlenstoffatomen oder durch ein oder mehrere Nitrogruppen substituierten Phenylrest bedeutet, oder $R_A$ eine Alkoxyrest mit 1 bis 5 Kohlenstoffatomen darstellt, oder R eine Gruppe —CHO oder —CO—NH—$C_6H_5$ bedeutet und $R_1$ einen Alkyoxyrest mit 1 bis 5 Kohlenstoffatomen darstellt, dadurch gekennzeichnet, daß man ein Produkt der Formel II

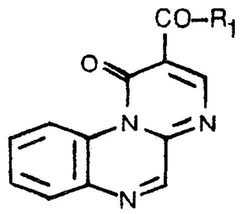

(II)

worin $R_1$ die angegebene Bedeutung besitzt, reduziert, um ein Produkt der Formel $I_a$

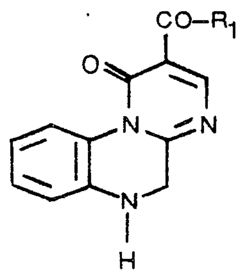

($I_a$)

entsprechend einem Produkt der Formel I, worin $R_1$ die angegebene Bedeutung besitzt und R ein Wasserstoffatom bedeutet, zu erhalten, und

*entweder* man das so erhaltene Produkt der Formel $I_a$ isoliert und gewünschtenfalls in ein Salz überführt

*oder* das Produkt der Formel $I_a$ mit einem Reagens umsetzt, das es ermöglicht, den Rest R, der von einem Wasserstoffatom verschieden ist, einzuführen, um ein Produkt der Formel $I_b$

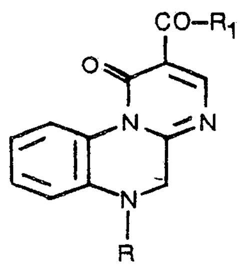

($I_b$)

entsprechend einem Produkt der Formel I, worin $R_1$ die angegebene Bedeutung besitzt und R die angegebene Bedeutung mit Ausnahme eines Wasserstoffatoms besitzt, zu erhalten, das man gewünschtenfalls in ein Salz überführen kann.

2. Verfahren gemäß Anspruch 1, dadurch gekennzeichnet, daß

(a) die Reduktion des Produkts der Formel II mit Hilfe von Wasserstoff in Gegenwart eines Katalysators, wie Palladium, durchgeführt wird,

(b) das Reagens, das es ermöglicht, den Rest R einzuführen, in dem Fall, bei dem R einen Alkylrest oder einen Rest —CO—$R_A$ bedeutet, aus einem Halogenid dieses Restes R, vorzugsweise einem Chlorid, besteht,

(c) das Reagens, das es ermöglicht, den Rest R einzuführen, in dem Fall, bei dem R eine Gruppe —CHO bedeutet, aus Ameisensäure in Gegenwart von Carbonyldiimidazol besteht,

(d) das Reagens, das es ermöglicht, den Rest R einzuführen, in dem Fall, bei dem R eine Gruppe —CO—NH—$C_6H_5$ bedeutet, Phenylisocyanat ist,

(e) das Reagens, das es ermöglicht, den Rest R einzurühren, in dem Fall, bei dem R einen Methylrest bedeutet, aus Formaldehyd besteht und der Umsetzung dieses letzteren sich eine Reduktion anschließt.

3. Verfahren gemäß Anspruch 1 oder 2, dadurch gekennzeichnet, daß man von einem Produkt der Formel II ausgeht, worin $R_1$ einen Ethoxyrest bedeutet.

4. Verfahren gemäß Anspruch 1, 2 oder 3, dadurch gekennzeichnet, daß das Reagens, das es ermöglicht, den Rest R einzuführen, derart ausgewählt wird, daß man einen Methylrest, einen Rest —CO—$R_A$, worin $R_A$ eine Methyl-, Ethyl, Isopropyl-, Cyclopropyl-, Cyclopentyl-, Phenyl-, p-Chlorphenyl- oder Ethoxygruppe bedeutet, oder eine Gruppe —CHO oder —CO—NH—$C_6H_5$ einführt.

5. Verfahren gemäß einem der Ansprüche 1 bis 4, dadurch gekennzeichnet, daß man den 1-Oxo-5,6-dihydro-1H-pyrimido[1,2-a]chinoxalin-2-carbonsäure-ethylester sowie dessen Salze herstellt.

6. Verfahren gemäß einem der Ansprüche 1 bis 4, dadurch gekennzeichnet, daß man den 6-Benzoyl-1-oxo-5,6-dihydro-1H-pyrimido[1,2-a]chinoxalin-2-carbonsäure-ethylester sowie dessen Salze herstellt.

7. Verfahren gemäß einem der Ansprüche 1 bis 4, dadurch gekennzeichnet, daß man den 1-Oxo-5,6-dihydro-1H-pyrimido[1,2-a]chinoxalin-2,6-carbonsäure-ethylester sowie dessen Salze herstellt.

**Claims for the Contracting States: BE CH DE FR IT LI LU NL SE**

1. Pyrimido-quinoxalines, as well as their salts, characterized in that they answer to the general formula I:

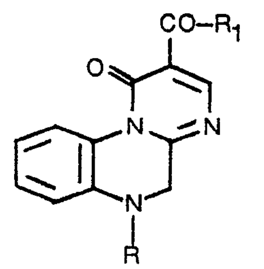

(I)

in which R represents a hydrogen atom, an alkyl radical containing from 1 to 5 carbon atoms, a —CO—$R_A$ radical in which $R_A$ represents a linear or branched alkyl group, containing from 1 to 6 carbon atoms, a cycloalkyl radical containing from 3 to 6 carbon atoms, a phenyl radical possibly substituted by one or more halogen atoms or by one or more alkoxy radicals containing from 1 to 3 carbon atoms, by one or more nitro radicals, or $R_A$ represents an alkoxy radical containing from 1 to 5 carbon atoms, or R represents a group —CHO or —CO—NH—$C_6H_5$, $R_1$ represents an alkyloxy radical containing from 1 to 5 carbon atoms.

2. Derivatives answering to the general formula I of Claim 1, as well as their salts, characterized in that in the said formula I, R has the significance already indicated and $R_1$ represents an ethoxy radical.

3. Derivatives answering to the general formula I of Claim 1, as well as their salts, characterized in that in the said formula I, R represents a hydrogen atom, a methyl radical, a —CO—$R_A$ radical in which $R_A$ represents a methyl, ethyl, isopropyl, cyclopropyl, cyclopentyl, phenyl, p-chlorophenyl or ethoxy group, or R represents a —CHO or —CO—NH—$C_6H_5$ group.

4. Ethyl 1-oxo-5,6-dihydro-1H-pyrimido[1,2-a]quinoxalin-2-carboxylate, as well as its salts.

5. Ethyl 6-benzoyl-1-oxo-5,6-dihydro-1H-pyrimido[1,2-a]quinoxalin-2-carboxylate, as well as its salts.

6. Ethyl 1-oxo-5,6-dihydro-1H-pyrimido[1,2-a]quinoxalin-2,6-dicarboxylate, as well as its salts.

7. Preparation process for products answering to the general formula I of Claim 1, as well as their salts, characterized in that a product with the formula II:

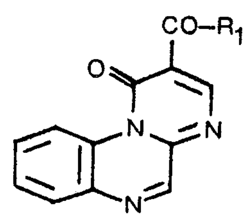

(II)

in which $R_1$ has the significance already indicated, is reduced so as to obtain a product with the formula $I_a$:

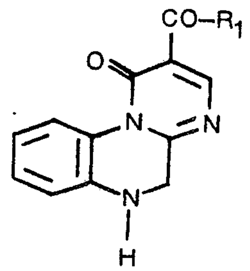

($I_a$)

corresponding to a product with the formula I, in which $R_1$ has the significance already indicated and R represents a hydrogen atom, and, which,

*either* is isolated, and the said product with the formula $I_a$ so obtained is salified if desired,

*or* the said product with the formula $I_a$ is made to react with a reagent enabling the radical R, which is different from a hydrogen atom, to be introduced so as to obtain a product with the formula $I_b$:

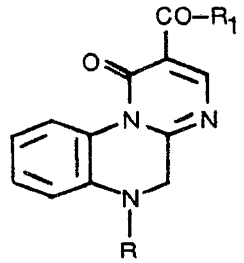

($I_b$)

corresponding to a product with the formula I, in which $R_1$ has the significance already indicated and R has the significance already indicated, with the exception of a hydrogen atom, which if desired can be salified.

8. Process according to Claim 7, characterized in that:

a) the reduction of the product with the formula II is carried out with hydrogen in the presence of a catalyst such as palladium,

b) the reagent which enables the radical R to be introduced in the case when R represents an alkyl radical or a —CO—$R_A$ radical is constituted by a halogenide of this R radical, preferably a chloride,

c) the reagent which enables the radical R to be introduced in the case when R represents a —CHO group is constituted by formic acid in the presence of carbonyl-diimidazole,

d) the reagent which enables the radical R to be introduced in the case when R represents a —CO—NH—$C_6H_5$ group is phenyl isocyanate,

e) the reagent which enables the radical R to be introduced in the case when R represents a methyl radical is constituted by formic aldehyde and the action of this latter is followed by a reduction.

9. Medicaments, characterized in that they are constituted by the new derivatives as defined by the formula I of Claim 1, as well as by their salts of addition with pharmaceutically acceptable acids.

10. Medicaments, characterized in that they are constituted by the new derivatives as defined in any one of the Claims 2 to 6, as well as by their salts of addition with pharmaceutically acceptable acids.

11. Pharmaceutical compositions, characterized in that they contain as active principle, at least one of the medicaments as defined in any one of the Claims 9 or 10.

**Claims for the Contracting State: AT**

1. Preparation process for new pyrimido-quinoxalines, as well as their salts, answering to the general formula I:

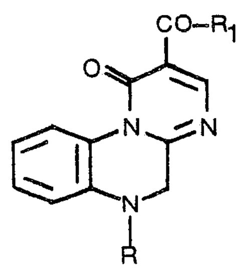

(I)

in which R represents a hydrogen atom, an alkyl radical containing from 1 to 5 carbon atoms, a —CO—$R_A$ radical in which $R_A$ represents a linear or branched alkyl group, containing from 1 to 6 carbon atoms, a cycloalkyl radical containing from 3 to 6 carbon atoms, a phenyl radical possibly substituted by one or more halogen atoms or by one or more alkoxy radicals containing from 1 to 3 carbon atoms, by one or more nitro radicals, or $R_A$ represents an alkoxy radical containing from 1 to 5 carbon atoms, or R represents a group —CHO or C—CO—NH—$C_6H_5$, $R_1$ represents an alkyloxy radical containing from 1 to 5 carbon atoms, characterized in that a product with the formula II:

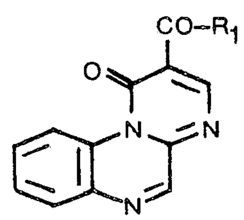

(II)

in which $R_1$ has the significance already indicated, is reduced so as to obtain a product with the formula $I_a$:

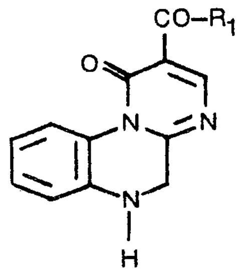

$(I_a)$

corresponding to a product with the formula I, in which $R_1$ has the significance already indicated and R represents a hydrogen atom, and, which,

*either* is isolated, and the said product with the formula $I_a$ so obtained is salified if desired,

*or* the said product with the formula $I_a$ is made to react with a reagent enabling the radical R, which is different from a hydrogen atom, to be introduced so as to obtain a product with the formula $I_b$:

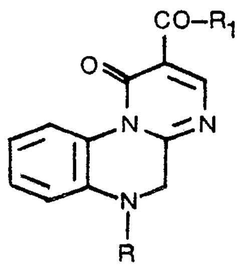

$(I_b)$

corresponding to a product with the formula I, in which $R_1$ has the significance already indicated and R has the significance already indicated, with the exception of a hydrogen atom, which if desired can be salified.

2. Process according to Claim 1, characterized in that:

a) the reduction of the product with the formula II is carried out with hydrogen in the presence of a catalyst such as palladium,

b) the reagent which enables the radical R to be introduced in the case when R represents an alkyl radical or a $—CO—R_A$ radical is constituted by a halogenide of this R radical, preferably a chloride,

c) the reagent which enables the radical R to be introduced in the case when R represents a —CHO group is constituted by formic acid in the presence of carbonyl-diimidazole,

d) the reagent which enables the radical R to be introduced in the case when R represents a $—CO—NH—C_6H_5$ group is phenyl isocyanate,

e) the reagent which enables the radical R to be introduced in the case when R represents a methyl radical is constituted by formic aldehyde and the action of this latter is followed by a reduction.

3. Process according to Claim 1 or 2, characterized in that at the start a product with the formula II is used in which $R_1$ represents an ethoxy radical.

4. Process according to Claim 1, 2 or 3, characterized in that the reagent which enables the radical R to be introduced is chosen in such a way that there is introduced a methyl radical, a $—CO—R_A$ radical in which $R_A$ represents a methyl, ethyl, isopropyl, cyclopropyl, cyclopentyl, phenyl, p-chlorophenyl or ethoxy group, or a —CHO or $—CO—NH—AC_6H_5$ group.

5. Process according to any one of the Claims 1 to 4, characterized in that ethyl 1-oxo-5,6-dihydro-1H-pyrimido[1,2-a]quinoxalin-2-carboxylate is prepared, as well as its salts.

6. Process according to any one of the Claims 1 to 4, characterized in that ethyl 6-benzoyl-1-oxo-5,6-dihydro-1H-pyrimido[1,2-a]quinoxalin-2-carboxylate is prepared, as well as its salts.

7. Process according to any one of the Claims 1 to 4, characterized in that ethyl 1-oxo-5,6-dihydro-1H-pyrimido[1,2-a]quinoxalin-2,6-dicarboxylate is prepared, as well as its salts.